(19) Europäisches Patentamt
European Patent Office
Office européen des brevets

(11) Publication number: **0 089 634**
**B1**

(12) **EUROPEAN PATENT SPECIFICATION**

(45) Date of publication of patent specification: **02.07.86**

(21) Application number: **83102667.9**

(22) Date of filing: **17.03.83**

(51) Int. Cl.[4]: **C 07 D 295/08,** C 07 D 207/27, C 07 D 211/76, A 61 K 31/495

(54) **Phenoxypropanolamine derivatives.**

(30) Priority: **18.03.82 JP 44089/82**

(43) Date of publication of application:
**28.09.83 Bulletin 83/39**

(45) Publication of the grant of the patent:
**02.07.86 Bulletin 86/27**

(84) Designated Contracting States:
**CH DE FR GB IT LI**

(56) References cited:
**EP-A-0 056 486**
**FR-A-2 113 878**
**FR-A-2 302 085**
**GB-A-1 290 490**

(73) Proprietor: **NIPPON SHINYAKU COMPANY, LIMITED**
**14, Kisshoin Nishinosho Monguchicho**
**Minami-ku Kyoto-shi Kyoto (JP)**

(72) Inventor: **Aoyagi, Yoshiaki**
**8-7-105 Uchidehama**
**Otsu-shi 520 (JP)**
Inventor: **Okubo, Takashi**
**Fuji Danchi A-9**
**Mikami Omi, Yasucho Shiga Pref. 520-23 (JP)**
Inventor: **Tomita, Toshio**
**635-13 Kamikasacho**
**Kusatsu-shi, Shiga Pref. 525 (JP)**
Inventor: **Nishida, Hiroshi**
**Room 1-309, 1-1 Higashitakenosatocho Oharano**
**Nishikyo-ku, Kyoto 610-11 (JP)**
Inventor: **Enomoto, Hiroshi**
**7-707, 26-3 Babamiba Hashiri**
**Nagaokakyoshi, Kyoto Pref. 611 (JP)**

(74) Representative: **Wey, Hans-Heinrich, Dipl.-Ing. et al**
**Patentanwälte Müller-Börner & Wey**
**Widenmayerstrasse 49**
**D-8000 München 22 (DE)**

**EP 0 089 634 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to novel 1-phenoxy-3-(4-phenylpiperazino)-2-propanol derivatives represented by the following general formula (I) and salts thereof which exhibit hypotensive action and are useful as pharmaceuticals.

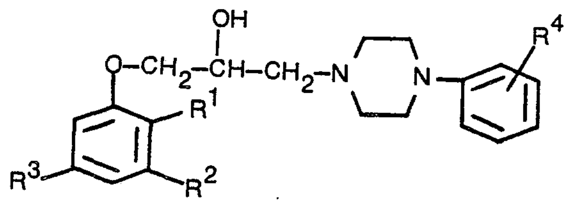

(I)

In the formula,

$R^1$ is hydrogen, halogen or methyl group; $R^2$ is pyrrolidino, piperidino, morpholino or perhydroazepino with or without carbonyl groups at alpha-position; $R^3$ is hydrogen, methyl group or ethoxycarbonyl group; $R^4$ is hydrogen, $C_1$—$C_2$alkyl group with or without alpha-ethoxy substituent(s), methoxy group, halogen, or acetyl group.

It has been known that 1-phenoxy-3-(4-phenylpiperazino)-2-propanol derivatives exhibit alpha-adrenergic blocking action, etc. (cf. Laid Open Japanese Patent Applications Sho-55-50950, 56-49360, 56-49361, 56-152420, 56-154431, 56-97227, 56-137174; German patents 2034278 and 3005287; Dutch patents 7011951 and 7308456). This prior art is also reflected in Chemical Abstracts 97:216231b, 92:215464q, 95:169204s and 83:163806v and in the US—PS 4,413,006.

The present inventors have conducted more extensive studies in order to find out more pharmaceutically effective compounds out of such 1-phenoxy-3-(4-phenylpiperazino)-2-propanol derivatives, found that novel compounds represented by the formula (I) in which a nitrogen-containing heterocyclic ring having a specific substituent is present at the 3-position of the phenoxy group exhibit alpha-adrenergic blocking action and beta-adrenergic blocking action and are applicable as excellent remedies for hypertension, and accomplished the present invention.

It has hitherto been known that alpha-adrenergic blocking agents are used as remedies effective for therapy or prevention of hypertension and for treatment of peripheral circulation insufficiencies such as Raynaud's disease. When such alpha-adrenergic blocking agents are solely administered, lowering of blood pressure due to vasodilating action surely takes place but, in some cases, reflex tachycardia is simultaneously induced. Of course it is possible to prevent reflex tachycardia due to blood pressure lowering by the simultaneous administration of beta-adrenergic blocking agents. However, in such a method, two kinds of drugs are to be used and the administration is troublesome.

Now the compound represented by the general formula (I) or pharmacologically acceptable salt thereof is unique as alpha, beta-adrenergic blocking agent since it possesses both strong alpha-adrenergic blocking agent action and beta-adrenergic action. Thus, the compounds represented by the general formula (I) and pharmacologically acceptable acids thereof are neither "prazosin which possesses strong alpha-adrenergic blocking action but do not possess beta-adrenergic blocking action" nor "labetalol which possesses both comparatively strong beta-adrenergic blocking action and weak alpha-adrenergic blocking action" but they possess "vasodilating action owing to their strong alpha-adrenergic action, surely hypertensive action by low doses, long acting effect, and excellent effect of inhibiting heart rate increase caused by hypotensive action because of their beta-adrenergic blocking action". In addition, those compounds do not exhibit antidiuretic action which is often observed in other adrenergic blocking agents and they are of low toxicity. In view of the above, the present invention compounds are very useful in medical therapy as therapeutic and preventing agents for hypertension.

Hypertension derives from various causes and its results in many diseases and make the diseases worse such as cerebrovascular disturbances (such as cerebral hemorrhage, cerebral infarction, cerebral arteriosclerosis and the like), ischemic heart diseases (such as mycocardial infarction, angina pectoris and the like), congestive heart failure and renal failure. Thus, in order to prevent such complications and to inhibit their progress to worse, it is a very important therapeutic method that high blood pressure is brought to near normal.

Compounds represented by the general formula (I) and pharmacologically acceptable salts thereof exhibit, as is obvious from the experimental results disclosed later, strong action to make blood pressure of animals lower and, due to their low toxicity, they are useful as pharmaceuticals for practical use.

Compounds according to the present invention may be manufactured by various routes. One of the representative routes is as follows:

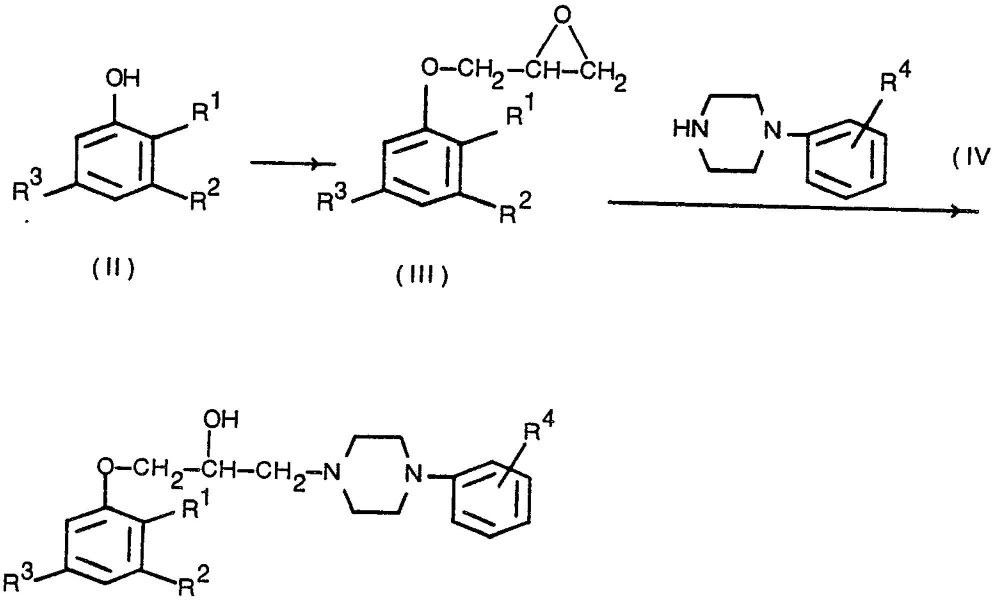

(II) (III) (IV)

(I)

In the above formulas, meanings of symbols $R^1$, $R^2$, $R^3$ and $R^4$ are the same as those already defined.

Thus, phenolic compound represented by the formula (II) is made to react with epihalogenohydrin compound in a suitable solvent in the presence of suitable carbonate agent such as potassium carbonate to give the corresponding epoxy compound (III). Then the epoxy compound (III) is made to react with an amine represented by the formula (IV) in a suitable solvent to afford the present invention compound (I). The term "suitable solvent" used hereinabove refers to a solvent which dissolves the starting materials even to a little extent and which will not react with the reaction agent and, therefore, it will not be restricted to specific solvents.

Examples as given hereinunder are given as mere exemplification and do not restrict reaction agents and reaction conditions thereto. The present invention compounds may also be prepared by other methods than them.

alpha, beta-Adrenergic blocking action and hypotensive action of representative compounds of the present invention compounds were tested and are given below.

1. alpha-Adrenergic action:

Test method is as follows. Thus, test compounds are given per os to ddY strain male mice (20 to 30 grams body weight; one group comprising five mice), then lethal dose of norepinephrine is injected to tail vein of the mice and, out of the survival numbers of the animals, the alpha-adrenergic blocking action of the test compounds is determined. In the Table 1, the doses where at least three mice out of five survived are given.

TABLE 1

| Compounds (Example Number) | Doses (mg/kg) |
|---|---|
| 1 | 1.0 |
| 3 | 10 |
| 7 | 2.5 |
| 12 | 1.0 |
| 13 | 1.0 |
| 16 | 1.0 |
| 17 | 10 |
| 22 | 5 |
| 23 | 1.0 |
| 24 | 1.0 |
| 27 | 1.0 |
| 28 | 2.5 |
| 29 | 2.5 |
| 33 | 2.5 |
| 35 | 2.5 |
| Prazosin | 10 |

2. beta-Adrenergic Blocking Action:

Test compounds are administered per os to unanesthetized male rats (250 grams body weight; five rats in one group) and, one hour thereafter, isoproterenol (0.01 mg/kg) is injected intravenously and the heart rate increase induced thereby is measured. This is compared with the control and the effect of inhibiting the increase by each test compound is calculated wherefrom beta-adrnergic blocking action of test compounds is evaluated. Table 2 shows the dose of test compounds which inhibits at least 50% or more of heart rate increase induced by isoproterenol.

TABLE 2

| Compounds (Example Numbers) | Doses (mg/kg) |
|---|---|
| 1 | 25 |
| 35 | 50 |
| 7 | 50 |
| 22 | 25 |
| 13 | 25 |
| Propranolol | 2.5 |

3. Hypotensive Action by Oral Administration:

Unanesthetized normal male rats (250 to 350 grams body weight; one group comprising 5 to 6 rats) are used and changes in blood pressure by oral administration of test compounds are observed by the indirect

tail cuff method. The results are given in Table 3. Blood pressure change rate (%) is calculated by the following expression:

$$\text{Blood pressure change rate (\%)} = \frac{\text{Blood pressure (mmHg) after Administration}}{\text{Blood pressure (mmHg) before Administration}} \times 100$$

TABLE 3

| Compounds (Example Number) | Doses (mg/kg) | Blood Pressure Change Rate (%) 0 | 120 | 240 | 350 min after administration |
|---|---|---|---|---|---|
| 1 | 50. | 100 | 86.2 | 78.1 | 70.4 |
| | 5 | 100 | 88.1 | 80.1 | 79.3 |
| 3 | 50 | 100 | 87.2 | 84.5 | 82.0 |
| 7 | 50 | 100 | 92.3 | 78.8 | 77.6 |
| | 5 | 100 | 92.6 | 83.3 | 80.6 |
| 12 | 50 | 100 | 75.6 | 70.6 | 70.2 |
| | 5 | 100 | 83.4 | 82.1 | 80.7 |
| 13 | 50 | 100 | 85.4 | 73.4 | 72.3 |
| | 5 | 100 | 88.7 | 79.2 | 78.4 |
| 16 | 50 | 100 | 87.9 | 78.3 | 76.2 |
| | 5 | 100 | 90.4 | 82.5 | 81.8 |
| 17 | 50 | 100 | 90.2 | 85.7 | 91.8 |
| 22 | 50 | 100 | 87.2 | 84.8 | 83.6 |
| 23 | 50 | 100 | 99.6 | 78.2 | 72.7 |
| | 5 | 100 | 97.4 | 81.4 | 80.5 |
| 24 | 50 | 100 | 86.9 | 76.5 | 78.4 |
| | 5 | 100 | 90.2 | 78.3 | 80.2 |
| 28 | 50 | 100 | 85.3 | 80.5 | 80.3 |
| | 5 | 100 | 89.6 | 82.2 | 82.7 |
| 29 | 50 | 100 | 88.1 | 77.6 | 75.2 |
| | 5 | 100 | 94.3 | 81.4 | 79.7 |
| 33 | 50 | 100 | 84.6 | 79.2 | 79.1 |
| | 5 | 100 | 92.3 | 82.5 | 80.4 |
| 35 | 50 | 100 | 85.6 | 81.1 | 82.3 |
| | 5 | 100 | 89.2 | 84.6 | 83.1 |
| Prazosin | 5 | 100 | 88.1 | 80.0 | 82.6 |

Then, taking a compound of Example 13 as a representative of the present invention compounds, we shall show its hypotensive action and heart rate exchange in natural hypertensive rats and its effects on urine volume and electrolytes in urine in renally hypertensive rats.

Hypotensive action and heart rate changes in neutral hypertensive rats.

Natural hypertensive rats showing not less than 180 mmHg systolic pressure are used. Under unanesthetized condition, a bloodless blood pressure measuring apparatus is used and the pressure is observed at tail artery. Thus, blood pressures and heart rates are measured with definite time intervals during the course of before and after administration of the compound. The results are given in Table 4.

TABLE 4

| Hours after Administration | 0.1 mg/kg by oral route | | 1.0 mg/kg by oral route | |
|---|---|---|---|---|
| | Systolic Pressure (mmHg) | Heart Rate (beats/min) | Systolic Pressure (mmHg) | Heart Rate (beats/min) |
| 0 | 191.3 ± 3.0 | 504 ± 10 | 190.2 ± 3.0 | 498 ± 13 |
| 1 | 178.0 ± 6.7 | 512 ± 3 | 168.0 ± 6.1 | 511 ± 14 |
| 3 | 168.7 ± 3.0 | 519 ± 10 | 161.8 ± 5.2 | 486 ± 18 |
| 6 | 165.3 ± 4.7 | 499 ± 14 | 137.4 ± 8.0 | 498 ± 15 |
| 9 | 172.3 ± 7.8 | 510 ± 8 | 174.6 ± 9.1 | 507 ± 3 |
| 12 | 180.5 ± 8.3 | 486 ± 14 | 170.8 ± 3.3 | 492 ± 9 |
| 24 | 189.7 ± 7.8 | 496 ± 18 | 189.0 ± 3.0 | 507 ± 13 |

(Figures in the table are mean value ± standard deviation)

Effects on Urine Volume and Electrolytes in Urine of Renally Hypertensive Rats.

Five renally hypertensive rats are made one group. Test compound is given orally and, simultaneously, 20 ml/kg of physiological saline solution (0.9% NaCL) is given orally. Effects of the test compound on urine volume and on electrolytes in urine are measured until 24 hours after administration. The results are given in Table 5.

TABLE 5

Results for the first 12 Hours:

| Compounds (Example Numbers) | Doses (mg/kg) per os | Urine Volume (ml) | Electrolytes $Na^+$ (mEq) | Electrolytes $K^+$ (mEq) | $Na^+/K^+$ |
|---|---|---|---|---|---|
| Control | 0 | 11.7 ± 1.5 | 1.58 ± 0.19 | 0.41 ± 0.07 | 4.55 ± 0.66 |
| 13 | 0.1 | 13.6 ± 1.3 | 1.13 ± 0.13 | 0.23 ± 0.03 | 5.68 ± 0.93 |
| Prazosin | 0.125 | 8.0 ± 0.7 | 0.74 ± 0.08 | 0.38 ± 0.04 | 2.18 ± 0.30 |

Results for the first 24 Hours:

| Compounds (Example Numbers) | Doses (mg/kg) per os | Urine Volume (ml) | Electrolytes $Na^+$ (mEq) | Electrolytes $K^+$ (mEq) | $Na^+/K^+$ |
|---|---|---|---|---|---|
| Control | 0 | 14.5 ± 1.7 | 1.97 ± 0.18 | 0.58 ± 0.18 | 3.90 ± 0.52 |
| 13 | 0.1 | 19.2 ± 2.6 | 1.65 ± 0.22 | 0.36 ± 0.04 | 5.23 ± 0.88 |
| Prazosin | 0.125 | 12.1 ± 1.0 | 1.31 ± 0.11 | 0.62 ± 0.05 | 2.23 ± 0.22 |

(Figures in the table are mean value ± deviation)

Acute Toxicity:

The present invention compounds themselves may be administered as remedies to animals including human beings or they may be administered as a pharmaceutical composition containing, for example, 0.1% to 99.5% or, more preferably, 1% to 80% of the present invention compound in phamaceutically-acceptable non-toxic and inert carriers.

Examples of the carriers applicable are one or more of solid, semi-solid or liquid diluents, fillers and other auxiliary agents for pharmaceutical purposes.

The pharmaceutical compositions are preferably administered in a form of unit dose.

The present invention pharmaceutical compositions may be administered orally, intrahistologically, locally (for example, percutaneously) or intrarectally. Of course preparations suitable for such a way of administration are applied and the most suitable example is an injection.

It is desired that the doses are regulated taking the age, body weight, and the like of the patients, administration route, kind and degree of the disease and the like into consideration. Usually the dose will be 5 to 300 mg per day and, more preferably 10 to 100 mg per day, of the present invention compound to adults. In some instances, it will be sufficient to use a lower dose and, on the other hand, it may be sometimes necessary to use a larger dose. Where a large amount is to be administered, it is desirable to give a divided dose several times a day.

Administration by the oral route may be conducted using a solid or liquid dose unit form such as, for example, powder, diluted powder, tablets, sugar coated tablets, capsules, granules, suspensions, liquids, syrups, drops, sublingal tablets and the like.

Powder can be manufactured by making the active substance into a suitable into a fine powder. Diluted powder can be manufactured by first making the active substance into a suitable fine powder and then by mixing with similarly powdered pharmaceutical carriers such as, for example, starch, mannitol, other edible hydrocarbon or the like. If necessary, additional auxiliaries such as, for example, tasting agent, preservatives, dispersing agents, coloring agents, perfumes, and the like may be added thereto.

Capsules may be manufactured, for example, as follows. Thus, powder or diluted powder as manufactured in the above description or granules as manufactured in the following description are filled in outer capsule such as, for example, geletine capsule. It is of course possible that lubricating or flowing agents such as, for example, colloidal silica, talc, magnesium stearate, calcium stearate, solid polyethylene glycol and the like are mixed with a preparation in powdery form followed by filling into capsules.

When disintegrating agents or solubilizing agents such as, for example, carboxymethyl cellulose, carboxymethyl cellulose calcium, hydroxypropyl cellulose with lower degree of substitution, calcium carbonate, sodium carbonate and the like are added thereto, it is possible to improve the efficiency of the pharmaceutical ingredients when the capsules are administered.

Fine powder of the present invention compounds may also be suspended and dispersed in vegetable oil, polyethylene glycol, glycerine, or surface active agents and then packed with gelatine sheets to prepare soft capsules.

Tablets can be manufactured by first preparing powdery mixture, then made into granules or slugs followed by addition of disintegrating agents or lubricating agents, and finally compressed into tablets.

Powdery mixtures may be prepared by mixing suitably powdered substances with the above-given diluents or bases and, if necessary, by mixing with combining agents (such as, for example, carboxymethyl cellulose sodium, alginates, geletin, polyvinyl pyrrolidone, polyvinyl alcohol, and the like), solubilization retarding agents (such as, for example, gelatin), reabsorption agents (such as, for example, quaternary salts) and/or adsorbing agents (such as, for example, bentonite, kaoline, dicalcium phosphate and the like).

Powdery mixtures can be made into granules by first mixing with combining agents such as, for example, syrup, starch paste, gum arabicum, cellulose solution or polymer solution to make wet granules and then passing through sieves under with pressure. Instead of granulating the powder as mentioned here, it is of course possible that the powder is first treated with a tablet manufacturing machine and the resulting slugs of uneven states are powdered into granules.

Granules prepared as above are, if desired, mixed with lubricating agents such as, for example, stearic acid, stearates, talc, mineral oil and the like so that their sticking each other can be prevented. Such lubricated mixture is then compressed into tablets. It is also possible that the pharmaceuticals are not subjected to a treatment for granules or slugs but they may be mixed with flowing and inert carriers followed by compressing into tablets directly. Transparent or semitransparent protective coating consisting of closed membrane of shellac, coating of sugar or polymer or brushing membrane comprising waxes may also be applicable.

Other orally acceptable preparations such as, for example, solutions, syrups, elixirs, and the like may be made into unit dose form containing a definite amount of pharmaceutical ingredient in a definite amount of the preparation.

Syrups are prepared by dissolving the compound into a suitable aqueous solution with an adequate perfume or taste. Elixers are prepared by the use of non-toxic alcoholic carriers. Suspensions are formulated by dispersing the compound into non-toxic carriers. If necessary, solubilizing agents or emulsifiers (such as, for example, ethoxylated isostearyl alcohols, polyoxyethylene sorbitol esters, and the like), preservatives, taste adhering agents (such as, for example, peppermint oil, saccharine, and the like) may also be added thereto.

If necessary, the unit dose preparations for oral administration may be made into microcapsules. Said formulations may also be coated or embedded in polymers or waxes so that elongation of acting hours or sustained-release action can be resulted.

Parenteral administration can be conducted by the use of liquid unit dose forms for hypodermic, intramuscular or intravenous injections such as, for example, by the use of a form of solution or suspension. They are prepared by suspending or dissolving definite amount of the compound into non-toxic liquid carrier suitable for injection such as, for example, aqueous or oily medium followed by sterilizing the resulting suspension or solution. Or, a definite amount of the compound is taken into a vial and then the said vial and the content therein are subjected to sterilization and close sealing. In order to dissolve or mix immediately before the administration, it is desired that preparatory vials or carriers may be added to the powdered or lyophilized active ingredients. Non-toxic salts or salt solutions may be added thereto to make the injection solution isotonic. It is further possible that stabilizer, preservatives, emulsifiers and the like may be added thereto.

Rectal administration can be conducted by the use of suppositories which are prepared by mixing the compound with low-melting and water soluble or water insoluble solid such as, for example, polyethylene glycol, cacao fat, higher esters (such as, for example, myristyl palmitate) and mixtures thereof.

To the pharmaceutical preparations of the present invention may be added other pharmaceuticals such as, for example, nitrites, beta-adrenergic blocking agents, diuretic hypotensive drugs, etc. or such pharmaceuticals may be given to patients together with the present invention preparations.

Manufacture method of the phenolic compound (II) used as a starting material for the compound (I) is given as hereunder for reference.

Reference Example

2-Chloropiperidinophenol

2-Chloro-3-piperidino-2-cyclohexenone (19.95 grams) and 9.19 grams of sodium acetate are dissolved in 500 ml of methanol and, with ice cooling and stirring, a solution of 17.25 grams of bromine in 200 ml of methanol is dropped thereinto during one hour. The mixture is stirred for another one hour at the same temperature, a solution of 26.2 grams of potassium hydroxide in 300 ml of methanol is dropped thereinto during 30 minutes, the mixture is stirred for one hour at a room temperature, concentrated to about 1/4 volume in vacuo, 400 ml of ice water is added thereto, then neutralized with 10% hydrochloric acid, separated matters therefrom are extracted with ether, the organic solvent layer is washed with saturated sodium chloride solution, dried with sodium sulfate, the solvent is evaporated therefrom, the residue is purified with silica gel chromatography, and recrystallized from n-hexane to give 14.71 grams (74.4%) of the product as colorless crystals of m.p. 91.5—92.0°C.

Starting from the corresponding (II), the following intermediates (III) are prepared.

1-(2-Chloro-5-methyl-3-(1-pyrrolidinyl)-phenoxy)-2,3-epoxypropane. Oily substance. Mass spectra: $M^+$ = 2.67. NMR ($CDCl_3$): δ 1.80—2.20 (m, 4H), 2.27 (s, 3H), 2.81 (s, 1H), 2.87 (d, 1H, J = 3Hz), 3.10—3.52 (m, 5H), 4.00 (dd, 2H, J = 4Hz and 11Hz), 4.28 (dd, 1H, J = 3.5 hz and 11 Hz), 6.20—6.50 (m, 2H).

1-(2-Chloro-5-methyl-3-piperidinophenoxy)-2,3-epoxypropane. Oily substance. NMR ($CDCl_3$): δ 1.30—2.10 (m, 6H), 2.26 (s, 3H), 2.78—3.15 (m, 6H), 3.16—3.50 (m, 1H), 3.95 (dd, 1H, J = 5 Hz and 12 Hz), 4.25 (dd, 1H, J = 4 Hz and 12 Hz), 6.45 (s, 2H).

1-(2-Chloro-2-(1-piperidinyl)-phenoxy-2,3-epoxypropane. Oily substance. Mass spectra: $M^+$ = 253. NMR ($CDCl_3$): δ 1.80—2.15 (m, 4H), 2.67—2.93 (m, 2H), 3.20—3.55 (m, 5H), 3.71—4.40 (m, 3H), 6.35—6.65 (m, 2H), 7.02 (t, 1H, J = 7.5 Hz).

1-(2-Chloro-5-ethoxycarbonyl-3-(1-pyrrolidinyl)-phenoxy)-2,3-epoxypropane. Oily substance (colorless). Mass spectra $M^+$ = 325. NMR ($CDCl_3$): δ 1.40 (t, 3H), J = 7Hz), 1.80—2.15 (m, 4H), 2.81 (s, 1H), 2.87 (d, 1H, J = 2 Hz), 3.22—3.60 (m, 5H), 3.80— 4.50 (m, 4H), 7.10 (d, 1H, J = 1.5 Hz), 7.21 (d, 1H, J = 1.5 Hz).

The present invention is further illustrated by the following examples.

Example 1

1-(3-Chloro-3-piperidinophenoxy)-3-(4-phenyl-1-piperazinyl)-2-propanol

(a) A mixture of 7.47 grams of 2-chloro-3-piperidinophenyl, 29.00 grams of epibromohydrin, 28.79 grams of potassium carbonate and 400 ml of acetonitrile is heated to reflux for twenty hours, insoluble matters are removed therefrom by filtration, the filtrate is entirely concentrated to dryness, and the residue is purified by a silica gel chromatography followed by recrystallization from n-hexane to give 8.0 grams (85%) of 1-(2-chloro-3-piperidinophenoxy)-2,3-epoxypropane as colorless crystals of m.p. 55—57°C. NMR ($CDCl_3$): δ 1.55—1.95 (m, 6H), 2.75—3.19 (m, 7H), 4.10 (dd, 1H, J = 11 hz and 14 Hz), 4.18 (dd, 1H, J = 11 Hz and 21 Hz), 6.54—6.77 (m, 2H). Elemental analysis calculated as $C_{14}H_{18}ClNO_2$: C 62.80, H 6.78, N 5.23, Cl 13.24; Found: C 62.61, H 6.87, N 5.08, Cl 13.15.

(b) 1-(2-Chloro-3-piperidinophenoxy)-2,3-epoxypropane (2.53 grams) obtained in (a) is heated to reflux for twelve hours with 4.60 grams of 1-phenylpiperazine and 200 ml of ethanol, the solvent is evaporated therefrom, the residue is purified by a silica gel chromatography, and recrystallized from ethanol to give 4.06 grams (86%) of the desired 1-(2-chloro-3-piperidinophenoxy)-3-(4-phenyl-1-piperazino)-2-propanol as colorless crystals of m.p. 107—108°C. NMR ($CDCl_3$): δ 1.65—2.00 (m, 6H), 2.50—3.32 (m, 15H), 4.01—4.28 (m, 3H), 6.51—7.41 (m, 8H). Elementary analysis calculated as $C_{24}H_{32}ClN_3O_2$: C 67.04, H 7.56, N 9.77, Cl 8.25; Found: C 66.90, H 7.70, N 9.47, Cl 8.21.

(c) 1-(2-Chloro-3-piperidinophenoxy)-3-(4-phenyl-1-piperazino)-2-propanol obtained in (b) is converted to its maleate (monohydrogen) and recrystallized from a mixture of ethanol and ether to give desired monohydrogen-maleate as colorless crystals of m.p. 150—152°C. NMR ($d_6$-DMSO): δ 1.51—1.80 (m, 6H), 2.80—3.15 (m, 4H), 3.20—3.51 (m, 10H), 4.01—4.20 (m, 2H), 4.21—4.51 (m, 1H), 6.10 (s, 2H), 6.65—7.43 (SH). Elementary analysis calculated as $C_{17}H_{27}ClN_2O_3\cdot CH_4H_4O_2\cdot \frac{1}{4}H_2O$: C 61.08, H 6.68, N 7.63, Cl 6.44; Found: C 61.10, H 7.06, N 7.38, Cl 6.20.

Compounds synthesized by the same method as given in Example 1 are listed in the following table.

TABLE 6

F: free compound

M: maleate

| Example No | $R^1$ | $R^2$ | $R^3$ | $R_4$ | Melting Point (°C) | (Salt) |
|---|---|---|---|---|---|---|
| 2 | Me | 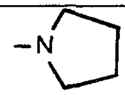 | H | H | 147~149 | M |
| 3 | Cl | " | H | H | 167~168 | M |
| 4 | Cl | " | Me | H | 166~167 | M |
| 5 | Cl | " | —$CO_2$Et | H | 165~166 | M |
| 6 | Cl | " | H | OEt<br>4-CHMe | 116~118 | F |
| 7 | Cl | " | H | 2-OMe | 152~153 | M |
| 8 | Me | " | H | 2-OMe | 152~153 | M |
| 9 | Cl | " | H | 4-OMe | 180~181 | M |
| 10 | Cl | " | H | 3-Cl | 163~165 | M |
| 11 | Cl | " | H | 4-COMe | 158~159 | M |
| 12 | Me | 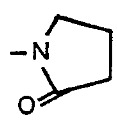 | H | H | 122~125 | F |
| 13 | Me | " | H | 2-OMe | 94~96 | F |
| 14 | H | 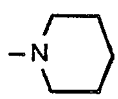 | H | H | 151~152 | M |
| 15 | Me | " | H | H | 159~161 | M |
| 16 | Br | " | H | H | 155~156 | M |
| 17 | Cl | " | Me | H | 167~168 | M |
| 18 | H | " | H | 2-Me | (glassy) | 3HCl |
| 19 | Me | " | H | 2-Me | 128~129 | M |
| 20 | Cl | " | H | 2-Me | 126~128 | M |
| 21 | H | " | H | 2-OMe | 117~120 | M |
| 22 | Me | " | H | 2-OMe | 136~138 | M |
| 23 | Cl | " | H | 2-OMe | 132~134 | M |

TABLE 6 (continued)

F: free compound
M: maleate

| Example No | $R^1$ | $R^2$ | $R^3$ | $R_4$ | Melting Point (°C) | (Salt) |
|---|---|---|---|---|---|---|
| 24 | Br | 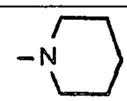 | H | 2-OMe | 122~125 | M |
| 25 | Cl | " | H | 4-OMe | 145~147 | M |
| 26 | Br | " | H | 4-OMe | 134~135 | M |
| 27 | Me | " | H | 3-Cl | 99~101 | M |
| 28 | Me | 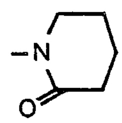 | H | H | 105~111 | F |
| 29 | H | " | H | H | 102~106 | 2HCl |
| 30 | H | " | H | 2-Me | 145~147 | F |
| 31 | H | 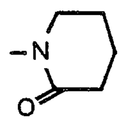 | H | 2-Me | 129~131 | F |
| 32 | H | " | H | 2-OMe | 129~131 | M |
| 33 | Me | " | H | 2-OMe | 106~111 | F |
| 34 | Cl | 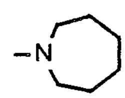 | H | H | 124~125 | M |
| 35 | Cl | 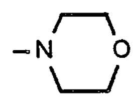 | H | H | 144~145 | M |
| 36 | Cl | " | H | Me | 144~146 | M |
| 37 | Cl | " | Me | H | 182~184 | M |

**Claim**

1-Phenoxy-3-(4-phenylpiperazino)-2-propanol derivatives represented by the following formula (I)

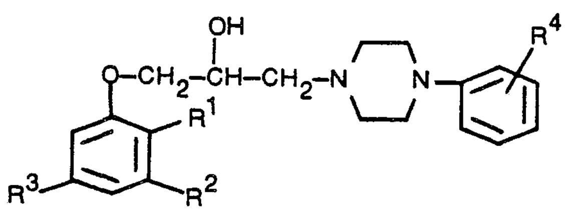

(I)

and pharmacologically accepable salts thereof in which $R^1$ is hydrogen, halogen or methyl group; $R^2$ is pyrrolidino, piperidino, morpholino or perhydroazepino with or without carbonyl group at alpha-position; $R^3$ is hydrogen, methyl group or ethoxycarbonyl group; $R^4$ is hydrogen, $C_1$—$C_2$alkyl group with or without alpha-ethoxy substituent(s), methoxy group, halogen, or acetyl group.

**Patentanspruch:**

1-Phenoxy-3-(4-phenylpiperazin)-2-propanolderivate der folgenden Formel (I)

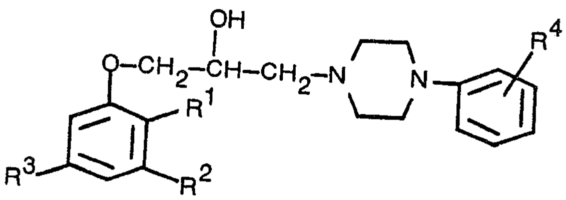

(I)

und ihre pharmazeutisch akzeptablen Salze, wobei $R^1$ ein Wasserstoffatom, ein Halogenatom oder die Methylgruppe; $R^2$ eine Pyrrolidin-, Piperidin-, Morpholin- oder Perhydroazepingruppe mit oder ohne eine Carbonylgruppe in α-Stellung; $R^3$ ein Wasserstoffatom, eine Methylgruppe oder Ethoxycarbonylgruppe; $R^4$ ein Wasserstoffatom, eine $C_1$—$C_2$-Alkylgruppe mit oder ohne einem oder mehreren α-Ethoxy-substituenten, eine Methoxygruppe, ein Halogenatom oder die Acetylgruppe ist.

**Revendication**

Dérivés du 1-phénoxy-3-(4-phénylpipérazino)-2-propanol représentés par la formule (I)

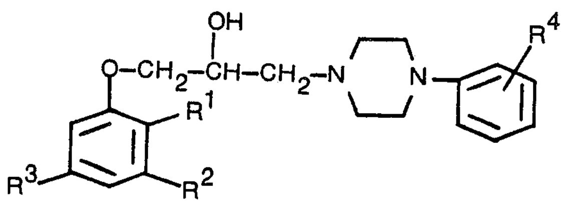

(I)

et leurs sels pharmaceutiquement acceptables dans lesquels $R^1$ est l'hydrogène, un halogène ou un groupe méthyle; $R^2$ est un groupe pyrrolidino, pipéridino, morpholino ou perhydroazépino avec ou sans groupe carbonyle en position alpha; $R^3$ est l'hydrogène, un groupe méthyle ou éthoxycarbonyle; $R^4$ est l'hydrogène, un groupe alkyle en $C_1$ à $C_2$ avec ou sans substituant(s) alpha-éthoxy, un groupe méthoxy, un halogène ou un groupe acétyle.